# EUROPEAN PATENT APPLICATION

(11) **EP 4 653 011 A1**
(43) Date of publication of application: **26.11.2025**
(21) Application number: 23917312.3
(22) Date of filing: 21.12.2023
(51) Int. Cl.: A61K 39/215, A61K 39/39, A61K 39/295, A61P 31/14, C12N 15/866, C12N 5/10, C12N 15/50, C07K 14/165

(54) **PREPARATION AND USE OF MRNA VACCINE AND RECOMBINANT PROTEIN SUBUNIT VACCINE AGAINST SARS-COV-2 OR MUTANT**

(30) Priority: 20.01.2023 CN 202310056799
(71) Applicant: WestVac Biopharma Co., Ltd., Chengdu, Sichuan 610000 (CN)
(72) Inventor: WEI, Xiawei, Chengdu, Sichuan 610065 (CN); LU, Guangwen, Chengdu, Sichuan 610065 (CN); CHENG, Ping, Chengdu, Sichuan 610065 (CN); YANG, Li, Chengdu, Sichuan 610065 (CN); LI, Jiong, Chengdu, Sichuan 610065 (CN); YANG, Jingyun, Chengdu, Sichuan 610065 (CN); WANG, Wei, Chengdu, Sichuan 610065 (CN); WEI, Yuquan, Chengdu, Sichuan 610065 (CN); WANG, Zhenling, Chengdu, Sichuan 610065 (CN); ZHAO, Zhiwei, Chengdu, Sichuan 610065 (CN); YANG, Jinliang, Chengdu, Sichuan 610065 (CN)
(74) Representative: Gulde & Partner
(86) International application number: PCT/CN2023/140745
(87) International publication number: WO 2024/152845

(57) **Abstract**

Provided in the present invention is a recombinant protein vaccine and/or an mRNA vaccine for preventing and/or treating infections of SARS-CoV-2 or a mutant thereof, and particularly provided is a method of using the mRNA vaccine and the recombinant protein vaccine. The vaccine can induce the generation of an antibody response and a cellular immune response in vivo to block the binding of the S protein of SARS-CoV-2 to the ACE2 receptor of host cells, so that the host resists coronavirus infections.

## Description

### TECHNICAL FIELD

The present invention relates to a drug and a combined drug against infection by SARS-CoV-2 or a variant thereof and a combined administration method therefor, and in particular, to an mRNA vaccine and/or a recombinant protein vaccine against infection by SARS-CoV-2 or a variant thereof and an administration method therefor, and belongs to the field of medicine.

### BACKGROUND

SARS-CoV-2 is a novel β coronavirus named by the World Health Organization. This virus is enveloped, and its particles are round or oval, often polymorphic, with a diameter of 60nm-140nm. Its genetic characteristics are significantly different from those of SARS-CoV and MERS-CoV, so the virus is a branch of novel coronavirus that has not been discovered in humans before. At present, there are five main types of SARS-CoV-2 variants: Alpha, Beta, Gamma, Delta, and Omicron, and Omicron variant strains are further divided into several sub-lines, such as BA.1, BA.2, BA.2.12.1, BA4 and BA5.

Main structural proteins of SARS-CoV-2 include spike (Spike, S) protein, envelope (Envelope, E) protein, membrane (Membrane, M) protein and nucleocapsid (Nucleocapsid, N) protein, among which the S protein plays a key role in virus infection and virulence and is often used as an antigen for vaccines. Because SARS-CoV-2 variant strains carry multiple mutation sites and the S protein of the virus also carries multiple mutation sites, the variant strains can escape antibodies elicited by vaccines of SARS-CoV-2 precursor strains to some extent, thereby diminishing the efficacy of SARS-CoV-2 vaccines or decreasing its protective immunity, and posing significant challenges to epidemic control efforts. Therefore, the development of vaccines against various variants of SARS-CoV-2 virus, especially broad-spectrum vaccines against various variants of SARS-CoV-2 virus, is of great significance for the prevention and control of SARS-CoV-2.

At present, more than 10 kinds of vaccines based on adenovirus vectors, mRNA and recombinant protein subunits have been approved all over the world. BNT162B2 (mRNA) from Pfizer Inc., ChAdOx1 nCoV-19 (adenovirus vector) from AstraZeneca Company, mRNA-1273 (mRNA) from Moderna Company, NVX-CoV2373 from Novavax Company (protein subunit) and AZD1222 (virus vector) from AstraZeneca Company are some typical vaccines. These SARS-CoV-2 vaccines effectively prevent diseases and fatal consequences caused by SARS-CoV-2 and its variants.

However, with the continuous evolution of SARS-CoV-2, the persistent emergence of novel variants has brought new challenges to the development of vaccines. Notably, dominant variant Omicron (such as B.1.1.529) has a large number of spike protein variants, is highly contagious and weakens the population protection given by the vaccines currently on the market. For example, the neutralization capacity against Omicron variant strains is reduced by 30 folds as compared to WA1/2020 bacterial strains at 2-4 weeks after vaccination with BNT162B2. Moreover, another study reveals that geometric mean titers (GMT) of 50% neutralizing antibodies between WA1/2020 strains and Omicron variants differ by 22 folds (1953 VS 89). Recently, a series of novel Omicron variants have emerged, such as BA.2, BA.3, BA.2.12.1, BA.4 and BA.5 (BA.4/5). Omicron BA.4/5 is dominant in South Africa and the United States. Although the spike protein mutation of BA.4/5 is equivalent to that of BA.2, the significantly enhanced neutralization escape capacity of BA.4/5 poses a more substantial barrier to vaccine development.

In view of the ongoing prevalence and continuous mutation of SARS-CoV-2, it is an important strategy to administer a third booster dose or develop new-generation vaccines against SARS-CoV-2. Existing researchers have demonstrated that three doses of BNT162B2 could significantly increase the neutralization capacity against Omicron. Similarly, homologous mRNA-1273 booster can also improve the effect of two doses of mRNA-1273 vaccines against Omicron variant. Although the third booster dose can enhance its neutralization capacity, its efficacy against BA.4/5 Omicron variant is still significantly reduced.

### SUMMARY

The present invention aims to solve one of technical problems existing in the prior art. Therefore, the purpose of the present invention is to provide a recombinant protein vaccine and an mRNA vaccine against infection by SARS-CoV-2 or a variant thereof and combined administration methods therefor.

The present invention provides an mRNA vaccine and a recombinant protein vaccine for preventing and/or treating infection by SARS-CoV-2 or a variant thereof, and two vaccines are preparations including the mRNA vaccine or the recombinant protein vaccine against infection by SARS-CoV-2 or the variant thereof as active components respectively.

The present invention further provides a composition against infection by SARS-CoV-2 or a variant thereof, and the composition includes a preparation with an mRNA vaccine and a recombinant protein vaccine as active components, wherein the mRNA vaccine includes mRNA constructed based on a nucleotide sequence shown in SEQ ID No.1 or SEQ ID No.2 sequence; and the recombinant protein vaccine includes a protein constructed based on an amino acid sequence shown in SEQ ID No.4 sequence.

The present invention further provides a combined drug for preventing and/or treating infection by SARS-CoV-2 or a variant thereof, and the combined drug includes an mRNA vaccine and a recombinant protein vaccine against SARS-CoV-2 or the variant thereof, wherein the mRNA vaccine and the recombinant protein vaccine are administered respectively, simultaneously or sequentially, the mRNA vaccine includes mRNA constructed based on a nucleotide sequence shown in SEQ ID No.1 sequence; and the recombinant protein vaccine includes a protein constructed based on an amino acid sequence shown in SEQ ID No.4 sequence.

The mRNA vaccine comprises a nucleotide sequence as shown in SEQ ID No. 1 or SEQ ID No. 2; or the nucleotide sequence is a variant that is homologous to SEQ ID No. 1 or SEQ ID No. 2, exhibits the same or similar biological activity, and is obtained by substitution and/or deletion and/or insertion of at least one amino acid in the sequences of SEQ ID No. 1 or SEQ ID No. 2; preferably, the mutant sequence is a variant obtained by substitution and/or deletion and/or insertion of 1 to 400 amino acids in the sequences of SEQ ID No. 1 or SEQ ID No. 2; more preferably, the mutant sequence is a variant obtained by substitution and/or deletion and/or insertion of 5 to 30 amino acids in the sequences of SEQ ID No. 1 or SEQ ID No. 2.

The amino acid sequence of the protein precursor of the recombinant protein vaccine is shown in the SEQ ID No.4 sequence; or the amino acid sequence is a variant that exhibits homology and same or similar biological activity with the SEQ ID No.4 sequence and is obtained by substitution and/or deletion and/or insertion of at least one amino acid in the SEQ ID No.4 sequence; preferably, an RBD sequence is a variant obtained by substitution and/or deletion and/or insertion of 1-400 amino acids in the SEQ ID No.4 sequence; and preferably, the RBD sequence is a variant obtained by substitution and/or deletion and/or insertion of 5-30 amino acids in the SEQ ID No.4 sequence.

Further, the mRNA vaccine, the recombinant protein vaccine or the combined drug is formulated as intramuscular injections, nasal drops, sprays, nasal sprays or inhalants. Preferably, the mRNA vaccine is formulated as the intramuscular injections, and the recombinant protein vaccine is formulated as the intramuscular injections and the nasal sprays.

Further, the recombinant protein vaccine includes a protein and/or a protein precursor against infection by SARS-CoV-2 or the variant thereof.

Further, the protein and/or the protein precursor includes a protein having at least one RBD sequence and at least one HR sequence derived from the S protein of SARS-CoV-2, and the sequence of the protein and/or the protein precursor is shown in SEQ ID No. 4 sequence.

Alternatively, the sequence is a variant that exhibits homology and same or similar biological activity with the SEQ ID No.4 sequence and is obtained by substitution and/or deletion and/or insertion of at least one amino acid in the SEQ ID No.4 sequence; preferably, an RBD sequence is a variant obtained by substitution and/or deletion and/or insertion of 1-400 amino acids in the SEQ ID No.4 sequence; and preferably, the RBD sequence is a variant obtained by substitution and/or deletion and/or insertion of 5-30 amino acids in the SEQ ID No.4 sequence.

Further, a protein formed by the RBD sequence and the HR sequence in the S protein spontaneously forms a trimer.

Further, a homologous amino acid sequence is selected from at least one of RBD sequence of Alpha, Beta, Gamma, Delta and Omicron.

Further, the protein precursor is a protein against infection by SARS-CoV-2 or the variant thereof, where the protein is linked to a signal peptide and/or a protein tag; preferably, the signal peptide includes a native signal peptide of S protein or a variant thereof and/or a human tPA signal peptide additionally appended before the native signal peptide; preferably, the protein tag is selected from at least one of a histidine tag, a thioredoxin tag, a glutathione transferase tag, a ubiquitin-like modifier protein tag, a maltose-binding protein tag, a c-Myc protein tag and an Avi tag protein tag; and more preferably, the protein tag is a Trx tag and/or a 6His tag.

Further, a protease recognition site for excising the protein tag is further linked to the protein against infection by SARS-CoV-2 or the variant thereof; and preferably, the protease is selected from at least one of enterokinase, TEV protease, thrombin, coagulation factor Xa, carboxypeptidase A and rhinovirus 3c protease.

Further, the amino acid sequence of the protein and/or the protein precursor is selected from SEQ ID No.4 sequence.

Further, the nucleotide sequence encoding the amino acid sequence is shown in SEQ ID No.5 sequence.

Further, the recombinant protein vaccine and/or the mRNA vaccine includes nucleic acids against infection by SARS-CoV-2 or the variant thereof.

Further, the nucleotide sequences of the nucleic acids are shown in SEQ ID No. 1, SEQ ID No. 2, and SEQ ID No. 5 sequences.

The present invention further provides a recombinant vector including a polynucleotide sequence, wherein that polynucleotide sequence is selected from at least one of SEQ ID No.1 and SEQ ID No.5 sequences.

Further, the recombinant vector is at least one of an insect baculovirus expression vector, a mammalian cell expression vector, an Escherichia coli expression vector and a yeast expression vector; preferably, the insect baculovirus expression vector is pFastBac1; preferably, the mammalian cell expression vector is CHO cell expression vector; further preferably, the CHO cell expression vector is pTT5 or FTP-002; preferably, the Escherichia coli expression vector is pET32a; and preferably, the yeast expression vector is pPICZaA.

The present invention further provides a host cell including the said recombinant vectors.

Further, the host cells are selected from at least one of insect cells, mammalian cells, Escherichia coli and yeast; preferably, the insect cells are selected from at least one of sf9 cells, sf21 cells and Hi5 cells; and preferably, the mammalian cells are either CHO cells or HEK293 cells.

The recombinant protein vaccine and/or the mRNA vaccine further include a pharmaceutically acceptable excipient or adjuvant component.

Further, the adjuvant component is an immunologic adjuvant; preferably, the immunologic adjuvant is selected from at least one of the following: squalene-based oil-in-water emulsion, aluminum salt, calcium salt, phytosaponin, phytopolysaccharide, monophosphate lipid A, muramyl dipeptide, muramyl tripeptide, bacterial toxin, GM-CSF cytokine, lipid and cationic liposomal material.

Further, the immunologic adjuvant satisfies at least one of the following: the squalene-based oil-in-water emulsion being MF59, the aluminum salt being selected from at least one of aluminum hydroxide and alum, the calcium salt being tricalcium phosphate, the phytosaponin being either QS-21 or ISCOM, the phytopolysaccharide being astragalus polysaccharide, the bacterial toxin being selected from at least one of recombinant cholera toxin and diphtheria toxin, the lipid being selected from at least one of the following: phosphatidylethanolamine, phosphatidylcholine, cholesterol, dioleoylphosphatidylethanolamine; the cationic liposome material being selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl-2,3-dioleyloxypropyl-2-(2-spermidinecarboxamido)ethyl ammonium trifluoroacetate, trimethyldodecylammonium bromide, trimethyltetradecylammonium bromide, trimethylhexadecylammonium bromide, dimethyldioctadecylammonium bromide, and CpG ODN.

The present invention further provides use of the above drugs, administered either alone or in combination with another drug, in preparation of drugs for preventing and/or treating infection by SARS-CoV-2 or a variant thereof.

Further, the SARS-CoV-2 variant strains include at least one of Alpha, Beta, Gamma, Delta and Omicron.

The present invention further provides a preparation method for the protein, and the method includes the following steps: culturing the host cell to express the required protein or precursor, and then recovering the required protein.

The present invention also provides a preparation method for the recombinant protein, and the method includes the following steps: constructing a recombinant vector including the polynucleotide, and immunizing a human body to produce the protein.

Further, the vector is selected from at least one of the following: mRNA, DNA vaccine, adenovirus, vaccinia Ankara virus and adeno-associated virus.

Beneficial effects: the present invention provides a protein and a vaccine against infection by SARS-CoV-2 or a variant thereof, and in particular to, an mRNA vaccine and a recombinant protein vaccine against infection by SARS-CoV-2 or the variant thereof, and the nucleotide sequences encoding the two vaccines are all selected from at least one of SEQ ID No.1, SEQ ID No.2 and SEQ ID No.5. According to the present invention, experiments show that the two vaccines being combined (that is, the recombinant protein vaccine being combined as a third heterologous booster with the two mRNA vaccines) can enhance the humoral and cellular immune response, showing higher anti-RBD IgG levels, stronger neutralizing antibodies and increased IFN-γ- secreting T cells. The experiments provide robust evidence for heterologous boosting strategies against SARS-CoV-2, demonstrating that a third heterologous protein subunit booster (RBD-HR/trimer vaccine) is a highly suitable candidate following two doses of the mRNA vaccines.
**SEQ ID No.1 Nucleotide sequence of S antigen of SARS-CoV-2 Delta mRNA vaccine:** 
ATGTTCGTGTTCCTGGTGCTGCTGCCCCTGGTGAGCAGCCAATGCGTGAACCTGAGAAC
AAGAACACAGCTGCCCCCCGCCTACACCAACAGCTTCACAAGAGGGGTCTACTACCCC
GACAAGGTGTTCAGAAGCAGCGTGCTGCACAGCACCCAAGACCTGTTCCTGCCCTTCTT
CTCCAACGTGACCTGGTTCCACGCCATCCACGTGAGCGGCACCAACGGCACCAAGAGA
TTCGACAACCCCGTGCTGCCCTTCAACGACGGCGTGTACTTCGCTAGCACCGAGAAGAG
CAACATCATCAGAGGCTGGATCTTCGGCACCACCCTGGACAGCAAGACACAGAGCCTG
CTGATTGTGAACAACGCTACCAACGTGGTGATCAAGGTGTGCGAGTTTCAGTTCTGCAA
CGACCCCTTCCTGGGGGTGTACTACCACAAAAACAACAAGAGCTGGATGGAGAGCGGC
GTGTACAGCAGCGCCAACAACTGCACCTTCGAGTACGTGAGCCAACCCTTCCTGATGGA
CCTGGAGGGCAAGCAAGGCAACTTCAAGAACCTCAGAGAGTTCGTGTTCAAGAACATC
GACGGCTACTTCAAGATCTACAGCAAGCACACCCCCATCAACCTGGTGAGAGACCTGCC
CCAAGGCTTCAGCGCCCTGGAGCCCCTGGTGGACCTGCCCATCGGCATCAACATCACAA
GATTTCAGACCCTGCTGGCCCTGCACAGAAGCTACCTGACACCCGGCGACAGCAGCAG
CGGGTGGACAGCCGGCGCTGCCGCTTACTACGTGGGCTACCTGCAGCCTAGAACCTTCC
TGCTGAAGTACAACGAGAACGGCACCATTACCGACGCCGTCGACTGCGCCCTGGACCC
CCTGAGCGAGACCAAGTGCACCCTGAAGAGCTTCACCGTGGAGAAGGGCATCTATCAG
ACAAGCAACTTCAGAGTGCAGCCCACCGAGAGCATCGTGAGATTCCCCAACATCACCA
ACCTGTGCCCCTTCGGCGAGGTGTTCAACGCCACAAGATTCGCTAGCGTGTACGCCTGG
AACAGAAAGAGAATCAGCAACTGCGTGGCCGACTACAGCGTGCTGTACAACAGCGCTA
GCTTCAGCACCTTCAAATGCTACGGGGTCAGCCCCACCAAGCTGAACGACCTGTGCTTC
 
ACCAACGTGTACGCCGACAGCTTCGTGATCAGAGGCGACGAGGTGAGACAGATCGCCC
CCGGGCAGACCGGCAAGATCGCCGACTACAACTACAAGCTGCCCGACGACTTCACCGG
CTGCGTGATCGCCTGGAACTCCAACAACCTGGATAGCAAGGTGGGCGGCAACTACAACT
ATAGATACAGACTGTTCAGAAAGAGCAACCTGAAGCCCTTCGAGAGAGACATCAGCAC
CGAGATCTACCAAGCCGGCAGCAAGCCCTGCAACGGCGTGGAGGGCTTCAACTGCTAC
TTCCCCCTGCAGAGCTACGGCTTTCAGCCCACCAACGGCGTGGGCTATCAGCCCTACAG
AGTGGTCGTGCTGAGCTTCGAGCTGCTGCACGCCCCTGCCACCGTGTGCGGCCCCAAG
AAGAGCACCAACCTGGTGAAGAACAAGTGCGTGAACTTCAACTTCAACGGCCTGACCG
GCACCGGCGTGCTGACCGAGAGCAACAAGAAGTTCCTCCCTTTTCAGCAGTTCGGCAG
AGACATCGCCGACACCACCGATGCCGTGAGAGACCCTCAGACCCTGGAGATCCTGGAC
ATCACCCCCTGCAGCTTTGGCGGCGTGAGCGTGATCACCCCCGGCACCAACACAAGCA
ACCAAGTGGCCGTGCTGTACCAAGGCGTGAACTGCACCGAGGTGCCCGTGGCCATCCA
CGCCGATCAGCTGACCCCCACCTGGAGAGTGTACAGCACCGGCAGCAACGTGTTTCAG
ACAAGAGCCGGCTGCCTGATCGGCGCCGAGCACGTGAACAACAGCTACGAGTGCGACA
TCCCCATCGGCGCCGGCATCTGCGCTAGCTATCAGACACAGACCAACAGCAGACGGAG
AGCTAGAAGCGTGGCTAGCCAAAGCATCATCGCCTACACCATGAGCCTGGGCGCCGAGA
ACAGCGTGGCCTACAGCAACAACAGCATCGCCATCCCCACCAACTTCACCATCAGCGTG
ACCACCGAGATCCTGCCCGTCTCCATGACCAAGACAAGCGTGGACTGCACCATGTACAT
CTGCGGCGACAGCACCGAGTGCAGCAACCTGCTCCTGCAGTACGGCAGCTTCTGCACA
CAGCTGAACAGAGCCCTGACCGGCATCGCCGTGGAGCAAGACAAGAACACCCAAGAG
GTGTTCGCCCAAGTGAAGCAGATCTACAAGACCCCCCCCATCAAGGACTTCGGCGGCTT
CAACTTCAGCCAAATCCTGCCCGACCCTAGCAAGCCTAGCAAGAGAAGCTTCATCGAGG
ACCTGCTGTTCAACAAGGTGACCCTGGCCGACGCCGGCTTCATCAAGCAGTACGGCGA
CTGCCTGGGCGACATCGCCGCTAGAGACCTGATCTGCGCTCAGAAGTTCAATGGCCTGA
CCGTCCTGCCCCCCCTGCTGACCGACGAGATGATCGCTCAGTACACAAGCGCCCTGCTG
GCCGGCACAATTACAAGCGGCTGGACATTCGGCGCTGGCGCCGCCCTGCAAATCCCCTT
CGCCATGCAGATGGCCTACAGATTCAACGGCATCGGCGTGACACAGAACGTGCTGTACG
AGAATCAGAAGCTGATCGCCAATCAGTTCAACAGCGCCATCGGCAAGATCCAAGACAG
CCTGAGCAGCACCGCTAGCGCCCTGGGCAAGCTGCAGAACGTGGTGAATCAGAACGCC
CAAGCCCTGAACACCCTGGTGAAGCAGCTGAGCAGCAACTTCGGCGCCATCAGCTCCG
TGCTGAACGATATCCTGAGCAGACTGGACCCCCCCGAGGCCGAGGTGCAGATCGACCG
 
GCTGATCACCGGCAGACTGCAGAGCCTGCAGACCTACGTGACACAGCAGCTGATCAGA
GCCGCCGAGATCAGAGCTAGCGCCAACCTGGCCGCCACCAAGATGAGCGAGTGCGTGC
TGGGGCAGAGCAAGAGAGTGGACTTCTGCGGCAAGGGCTACCACCTGATGAGCTTCCC
TCAGAGCGCCCCCCACGGCGTGGTGTTCCTGCACGTGACCTACGTGCCCGCCCAAGAG
AAGAACTTCACCACCGCCCCCGCCATCTGCCACGACGGCAAGGCCCACTTCCCTAGAG
AGGGCGTGTTCGTGAGCAACGGCACCCACTGGTTCGTGACACAGAGAAACTTCTACGA
GCCTCAGATCATCACCACCGACAACACCTTCGTGAGCGGCAACTGCGACGTGGTGATCG
GCATTGTGAACAATACAGTCTACGACCCTCTGCAGCCCGAGCTGGACAGCTTCAAGGAG
GAGCTGGACAAGTACTTCAAGAATCACACAAGCCCCGACGTGGACCTGGGGGACATCA
GCGGCATCAACGCTAGCGTGGTGAACATTCAGAAGGAGATCGACAGACTGAACGAGGT
GGCCAAGAACCTGAACGAGAGCCTGATCGACCTGCAAGAGCTGGGCAAGTACGAGCA
GTACATCAAGTGGCCCTGGTACATCTGGCTGGGCTTCATCGCCGGCCTGATCGCCATCGT
GATGGTGACCATCATGCTGTGCTGCATGACAAGCTGCTGCAGCTGCCTGAAGGGCTGCT
GCTCCTGCGGCAGCTGCTGCAAGTTCGACGAGGACGACAGCGAGCCCGTGCTGAAGGG
CGTGAAGCTGCACTACACCTGA
**SEQ ID No. 2 mRNA sequence of S antigen of SARS-CoV-2 Delta mRNA vaccine:** 
AUGUUCGUGUUCCUGGUGCUGCUGCCCCUGGUGAGCAGCCAAUGCGUGAACCUGAG
AACAAGAACACAGCUGCCCCCCGCCUACACCAACAGCUUCACAAGAGGGGUCUACUA
CCCCGACAAGGUGUUCAGAAGCAGCGUGCUGCACAGCACCCAAGACCUGUUCCUGCC
CUUCUUCUCCAACGUGACCUGGUUCCACGCCAUCCACGUGAGCGGCACCAACGGCAC
CAAGAGAUUCGACAACCCCGUGCUGCCCUUCAACGACGGCGUGUACUUCGCUAGCAC
CGAGAAGAGCAACAUCAUCAGAGGCUGGAUCUUCGGCACCACCCUGGACAGCAAGA
CACAGAGCCUGCUGAUUGUGAACAACGCUACCAACGUGGUGAUCAAGGUGUGCGAG
UUUCAGUUCUGCAACGACCCCUUCCUGGGGGUGUACUACCACAAAAACAACAAGAG
CUGGAUGGAGAGCGGCGUGUACAGCAGCGCCAACAACUGCACCUUCGAGUACGUGA
GCCAACCCUUCCUGAUGGACCUGGAGGGCAAGCAAGGCAACUUCAAGAACCUCAGA
GAGUUCGUGUUCAAGAACAUCGACGGCUACUUCAAGAUCUACAGCAAGCACACCCC
CAUCAACCUGGUGAGAGACCUGCCCCAAGGCUUCAGCGCCCUGGAGCCCCUGGUGGA
CCUGCCCAUCGGCAUCAACAUCACAAGAUUUCAGACCCUGCUGGCCCUGCACAGAAG
CUACCUGACACCCGGCGACAGCAGCAGCGGGUGGACAGCCGGCGCUGCCGCUUACUA
 
CGUGGGCUACCUGCAGCCUAGAACCUUCCUGCUGAAGUACAACGAGAACGGCACCA
UUACCGACGCCGUCGACUGCGCCCUGGACCCCCUGAGCGAGACCAAGUGCACCCUGA
AGAGCUUCACCGUGGAGAAGGGCAUCUAUCAGACAAGCAACUUCAGAGUGCAGCCC
ACCGAGAGCAUCGUGAGAUUCCCCAACAUCACCAACCUGUGCCCCUUCGGCGAGGUG
UUCAACGCCACAAGAUUCGCUAGCGUGUACGCCUGGAACAGAAAGAGAAUCAGCAA
CUGCGUGGCCGACUACAGCGUGCUGUACAACAGCGCUAGCUUCAGCACCUUCAAAU
GCUACGGGGUCAGCCCCACCAAGCUGAACGACCUGUGCUUCACCAACGUGUACGCCG
ACAGCUUCGUGAUCAGAGGCGACGAGGUGAGACAGAUCGCCCCCGGGCAGACCGGC
AAGAUCGCCGACUACAACUACAAGCUGCCCGACGACUUCACCGGCUGCGUGAUCGCC
UGGAACUCCAACAACCUGGAUAGCAAGGUGGGCGGCAACUACAACUAUAGAUACAG
ACUGUUCAGAAAGAGCAACCUGAAGCCCUUCGAGAGAGACAUCAGCACCGAGAUCU
ACCAAGCCGGCAGCAAGCCCUGCAACGGCGUGGAGGGCUUCAACUGCUACUUCCCCC
UGCAGAGCUACGGCUUUCAGCCCACCAACGGCGUGGGCUAUCAGCCCUACAGAGUG
GUCGUGCUGAGCUUCGAGCUGCUGCACGCCCCUGCCACCGUGUGCGGCCCCAAGAAG
AGCACCAACCUGGUGAAGAACAAGUGCGUGAACUUCAACUUCAACGGCCUGACCGG
CACCGGCGUGCUGACCGAGAGCAACAAGAAGUUCCUCCCUUUUCAGCAGUUCGGCA
GAGACAUCGCCGACACCACCGAUGCCGUGAGAGACCCUCAGACCCUGGAGAUCCUGG
ACAUCACCCCCUGCAGCUUUGGCGGCGUGAGCGUGAUCACCCCCGGCACCAACACAA
GCAACCAAGUGGCCGUGCUGUACCAAGGCGUGAACUGCACCGAGGUGCCCGUGGCC
AUCCACGCCGAUCAGCUGACCCCCACCUGGAGAGUGUACAGCACCGGCAGCAACGUG
UUUCAGACAAGAGCCGGCUGCCUGAUCGGCGCCGAGCACGUGAACAACAGCUACGA
GUGCGACAUCCCCAUCGGCGCCGGCAUCUGCGCUAGCUAUCAGACACAGACCAACAG
CAGACGGAGAGCUAGAAGCGUGGCUAGCCAAAGCAUCAUCGCCUACACCAUGAGCC
UGGGCGCCGAGAACAGCGUGGCCUACAGCAACAACAGCAUCGCCAUCCCCACCAACU
UCACCAUCAGCGUGACCACCGAGAUCCUGCCCGUCUCCAUGACCAAGACAAGCGUGG
ACUGCACCAUGUACAUCUGCGGCGACAGCACCGAGUGCAGCAACCUGCUCCUGCAGU
ACGGCAGCUUCUGCACACAGCUGAACAGAGCCCUGACCGGCAUCGCCGUGGAGCAAG
ACAAGAACACCCAAGAGGUGUUCGCCCAAGUGAAGCAGAUCUACAAGACCCCCCCCA
UCAAGGACUUCGGCGGCUUCAACUUCAGCCAAAUCCUGCCCGACCCUAGCAAGCCUA
GCAAGAGAAGCUUCAUCGAGGACCUGCUGUUCAACAAGGUGACCCUGGCCGACGCC
GGCUUCAUCAAGCAGUACGGCGACUGCCUGGGCGACAUCGCCGCUAGAGACCUGAU
 
CUGCGCUCAGAAGUUCAAUGGCCUGACCGUCCUGCCCCCCCUGCUGACCGACGAGAU
GAUCGCUCAGUACACAAGCGCCCUGCUGGCCGGCACAAUUACAAGCGGCUGGACAU
UCGGCGCUGGCGCCGCCCUGCAAAUCCCCUUCGCCAUGCAGAUGGCCUACAGAUUCA
ACGGCAUCGGCGUGACACAGAACGUGCUGUACGAGAAUCAGAAGCUGAUCGCCAAU
CAGUUCAACAGCGCCAUCGGCAAGAUCCAAGACAGCCUGAGCAGCACCGCUAGCGCC
CUGGGCAAGCUGCAGAACGUGGUGAAUCAGAACGCCCAAGCCCUGAACACCCUGGU
GAAGCAGCUGAGCAGCAACUUCGGCGCCAUCAGCUCCGUGCUGAACGAUAUCCUGA
GCAGACUGGACCCCCCCGAGGCCGAGGUGCAGAUCGACCGGCUGAUCACCGGCAGAC
UGCAGAGCCUGCAGACCUACGUGACACAGCAGCUGAUCAGAGCCGCCGAGAUCAGA
GCUAGCGCCAACCUGGCCGCCACCAAGAUGAGCGAGUGCGUGCUGGGGCAGAGCAA
GAGAGUGGACUUCUGCGGCAAGGGCUACCACCUGAUGAGCUUCCCUCAGAGCGCCCC
CCACGGCGUGGUGUUCCUGCACGUGACCUACGUGCCCGCCCAAGAGAAGAACUUCAC
CACCGCCCCCGCCAUCUGCCACGACGGCAAGGCCCACUUCCCUAGAGAGGGCGUGUU
CGUGAGCAACGGCACCCACUGGUUCGUGACACAGAGAAACUUCUACGAGCCUCAGA
UCAUCACCACCGACAACACCUUCGUGAGCGGCAACUGCGACGUGGUGAUCGGCAUU
GUGAACAAUACAGUCUACGACCCUCUGCAGCCCGAGCUGGACAGCUUCAAGGAGGA
GCUGGACAAGUACUUCAAGAAUCACACAAGCCCCGACGUGGACCUGGGGGACAUCA
GCGGCAUCAACGCUAGCGUGGUGAACAUUCAGAAGGAGAUCGACAGACUGAACGAG
GUGGCCAAGAACCUGAACGAGAGCCUGAUCGACCUGCAAGAGCUGGGCAAGUACGA
GCAGUACAUCAAGUGGCCCUGGUACAUCUGGCUGGGCUUCAUCGCCGGCCUGAUCG
CCAUCGUGAUGGUGACCAUCAUGCUGUGCUGCAUGACAAGCUGCUGCAGCUGCCUG
AAGGGCUGCUGCUCCUGCGGCAGCUGCUGCAAGUUCGACGAGGACGACAGCGAGCC
CGUGCUGAAGGGCGUGAAGCUGCACUACACCUGA
SEQ ID No. 3 Amino acid sequence of S antigen of SARS-CoV-2 Delta mRNA vaccine: 
MFVFLVLLPLVSSQCVNLRTRTQLPPAYTNSFTRGVYYPDKVFRSSVLHSTQDLFLPFFSNVT
WFHAIHVSGTNGTKRFDNPVLPFNDGVYFASTEKSNIIRGWIFGTTLDSKTQSLLIVNNATN
VVIKVCEFQFCNDPFLGVYYHKNNKSWMESGVYSSANNCTFEYVSQPFLMDLEGKQGNF
KNLREFVFKNIDGYFKIYSKHTPINLVRDLPQGFSALEPLVDLPIGINITRFQTLLALHRSYLT
PGDSSSGWTAGAAAYYVGYLQPRTFLLKYNENGTITDAVDCALDPLSETKCTLKSFTVEKG
IYQTSNFRVQPTESIVRFPNITNLCPFGEVFNATRFASVYAWNRKRISNCVADYSVLYNSASFS
 
TFKCYGVSPTKLNDLCFTNVYADSFVIRGDEVRQIAPGQTGKIADYNYKLPDDFTGCVIAW
NSNNLDSKVGGNYNYRYRLFRKSNLKPFERDISTEIYQAGSKPCNGVEGFNCYFPLQSYGF
QPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQGVNCTEVP
VAIHADQLTPTWRVYSTGSNVFQTRAGCLIGAEHVNNSYECDIPIGAGICASYQTQTNSRRR
ARSVASQSIIAYTMSLGAENSVAYSNNSIAIPTNFTISVTTEILPVSMTKTSVDCTMYICGDST
ECSNLLLQYGSFCTQLNRALTGIAVEQDKNTQEVFAQVKQIYKTPPIKDFGGFNFSQILPDPS
KPSKRSFIEDLLFNKVTLADAGFIKQYGDCLGDIAARDLICAQKFNGLTVLPPLLTDEMIAQ
YTSALLAGTITSGWTFGAGAALQIPFAMQMAYRFNGIGVTQNVLYENQKLIANQFNSAIGKI
QDSLSSTASALGKLQNVVNQNAQALNTLVKQLSSNFGAISSVLNDILSRLDPPEAEVQIDRLI
TGRLQSLQTYVTQQLIRAAEIRASANLAATKMSECVLGQSKRVDFCGKGYHLMSFPQSAPH
GVVFLHVTYVPAQEKNFTTAPAICHDGKAHFPREGVFVSNGTHWFVTQRNFYEPQIITTDN
TFVSGNCDVVIGIVNNTVYDPLQPELDSFKEELDKYFKNHTSPDVDLGDISGINASVVNIQK
EIDRLNEVAKNLNESLIDLQELGKYEQYIKWPWYIWLGFIAGLIAIVMVTIMLCCMTSCCSC
LKGCCSCGSCCKFDEDDSEPVLKGVKLHYT*
**SEQ ID No.4 SARS-CoV-2 Delta RBD-HR/trimer protein precursor sequence (signal peptide-Trx tag-6His tag-EK enzyme digestion site-RBD sequence-HR1 sequence - HR2 sequence):** 
MLLVNQSHQGFNKEHTSKMVSAIVLYVLLAAAAHSAFAADSIHIKDSDDLKNRLAEAGDK
LVVIDFMATWCGPCKMIGPKLDEMANEMSDSIVVLKVDVDECEDIATEYNINSMPTFVFVK
NSKKIEEFSGANVDKLRNTIIKLKLAGSGSGHMHHHHHHSSGDDDDKVQPTESIVRFPNITN
LCPFGEVFNATRFASVYAWNRKRISNCVADYSVLYNSASFSTFKCYGVSPTKLNDLCFTNVY
ADSFVIRGDEVRQIAPGQTGKIADYNYKLPDDFTGCVIAWNSNNLDSKVGGNYNYRYRLF
RKSNLKPFERDISTEIYQAGSKPCNGVEGFNCYFPLQSYGFQPTNGVGYQPYRVVVLSFELL
HAPATVCGPKKSTNLVKNKSVNFNFNGLYENQKLIANQFNSAIGKIQDSLSSTASALGKLQD
VVNQNAQALNTLVKQLKNHTSPDVDLGDISGINASVVNIQKEIDRLNEVAKNLNESLIDLQ
EL
**SEQ ID No. 5 nucleotide sequence encoding SEQ ID No. 4:**

ATGCTACTAGTAAATCAGTCACACCAAGGCTTCAATAAGGAACACACAAGCAAGATGGT
GAGCGCTATCGTGCTGTACGTCCTGCTGGCCGCTGCTGCTCACAGCGCTTTCGCTGCTG
ACTCCATCCACATCAAGGACAGCGACGACCTGAAGAACCGTCTGGCCGAGGCCGGTGA

CAAGCTGGTCGTCATCGACTTCATGGCCACTTGGTGCGGTCCTTGCAAGATGATCGGCC
CTAAGCTGGACGAGATGGCTAACGAGATGTCCGACAGCATCGTGGTCCTGAAGGTGGA
CGTCGACGAGTGCGAGGACATCGCCACCGAATACAACATCAACAGCATGCCCACCTTCG
TGTTCGTGAAGAACAGCAAGAAGATCGAGGAATTTTCCGGCGCTAACGTCGACAAGCT
GCGTAACACCATCATCAAGCTGAAGCTGGCCGGCTCCGGCTCCGGCCACATGCATCACC
ACCACCACCATTCCTCCGGTGACGACGACGACAAGGTGCAGCCAACCGAATCTATCGTC
AGATTCCCAAACATCACTAACCTGTGCCCTTTCGGAGAGGTGTTCAACGCTACCAGGTT
CGCCAGCGTCTACGCTTGGAACCGCAAGCGTATCAGCAACTGCGTCGCCGACTACTCTG
TGCTGTACAACTCCGCTAGCTTCTCTACTTTCAAGTGCTACGGCGTGTCACCTACCAAGC
TGAACGACCTGTGCTTCACTAACGTCTACGCCGACTCCTTCGTGATCCGCGGAGACGAA
GTCCGTCAGATCGCTCCTGGACAGACCGGAAAGATCGCTGACTACAACTACAAGCTGCC
AGACGACTTCACTGGCTGCGTGATCGCTTGGAACTCAAACAACCTGGACTCCAAGGTC
GGTGGCAACTACAACTACCGGTACAGGCTGTTCAGAAAGTCAAACCTGAAGCCTTTCG
AGCGCGACATCTCAACCGAAATCTACCAGGCTGGTTCCAAGCCCTGCAACGGTGTGGA
GGGCTTCAACTGCTACTTCCCCCTGCAGTCCTACGGTTTCCAGCCAACCAACGGAGTCG
GTTACCAGCCTTACCGTGTGGTCGTGCTGAGCTTCGAACTGCTCCACGCTCCTGCTACTG
TGTGCGGTCCCAAGAAGTCTACTAACCTGGTCAAAAACAAAAGCGTCAACTTCAACTTC
AACGGTCTGTACGAGAACCAGAAGCTGATCGCTAACCAGTTCAACAGCGCCATCGGCA
AGATCCAGGACTCACTGTCATCCACTGCCTCCGCTCTGGGAAAGCTGCAGGACGTCGTG
AACCAGAACGCCCAGGCTCTGAACACCCTGGTCAAGCAGCTGAAGAACCACACCTCTC
CTGACGTCGACCTGGGCGACATCAGCGGAATCAACGCTTCTGTCGTGAACATCCAGAAG
GAGATCGACCGCCTGAACGAAGTGGCCAAGAACCTGAACGAATCCCTGATTGACCTCC
AAGAACTCTAA

### Terms and abbreviations:

Monophosphate lipid a (MPL); squalene-based oil-in-water emulsion (MF59), recombinant cholera toxin (rCTB), astragalus polysaccharide (APS), phosphatidylethanolamine (PE), phosphatidylcholine (PC), cholesterol (Chol), dioleoyl phosphatidylethanolamine (DOPE)(2,3-dioleoxypropyl) trimethyl ammonium chloride (DOTAP), N-[N- trimethylamine chloride (DOTMA), cationic cholesterol (DC-Chol), dimethyl trifluoroacetate-2,3-dioleoxypropyl-2-(2-spermamido) ethylammonium (DOSPA), trimethyl dodecyl ammonium bromide (DDAB), trimethyl tetradecyl ammonium bromide (TTAB), trimethyl cetylammonium bromide (CTAB), dimethyl didoctadecyl ammonium bromide (DDAB), CpG ODN (A nucleotide sequence with unmethylated cytosine and guanine dinucleotides as the core sequence, artificially synthesized CpG).

### BRIEF DESCRIPTION OF THE DRAWINGS

FIG. 1 shows a structural diagram of an mRNA sequence in Embodiment 1;
FIG. 2 shows a schematic diagram of preparation of an mRNA vaccine in Embodiment 1;
FIG. 3 shows a schematic structural diagram of a recombinant protein in Embodiment 1;
FIG. 4 shows a schematic diagram of immunization program and grouping in Test Example 1;
FIG. 5 shows test results of anti-RBD specific antibodies in mice on days 0, 21, 42 post-vaccination in Test Example 2;
FIG. 6 shows test results of anti-RBD specific antibodies in mice on days 0, 21, 111 post-vaccination in Test Example 2;
FIG. 7 shows levels of pseudovirus neutralizing antibodies in mice on days 0, 21, 42 post-vaccination in Test Example 3;
FIG. 8 shows levels of pseudovirus neutralizing antibodies in mice on days 0, 21, 111 post-vaccination in Test Example 3;
FIG. 9 shows levels of pseudovirus neutralizing antibodies against Omicron and a variant strain thereof in mice on days 0, 21, 111 post-vaccination in Test Example 3;
FIG. 10 shows flow cytometry plots of serum blocking assays of mice on days 0, 21, 42 post-vaccination in Test Example 4;
FIG. 11 shows statistical results of serum blocking assays of mice on days 0, 21, 42 post-vaccination in Test Example 4;
FIG. 12 shows flow cytometry plots of serum blocking assays of mice on days 0, 21, 111 post-vaccination in Test Example 4;
FIG. 13 shows statistical results of serum blocking assays of mice on days 0, 21, 111 post-vaccination in Test Example 4;
FIG. 14 shows typical flow cytometry plots of CD4⁺ (upper figure) and CD8⁺ (lower figure) memory T cells generated by RBD-specific IL-4 in Test Example 5;
FIG. 15 shows a level result graph of IL-4 in a cell supernatant analyzed via ELISA in Test Example 5;
FIG. 16 shows typical flow cytometry plots of CD4⁺ (upper figure) and CD8⁺ (lower figure) memory T cells generated by RBD-specific IFN-γ in Test Example 5;
FIG. 17 shows a level graph of IFN-γ in a cell supernatant analyzed via ELISA in Test Example 5;
FIG. 18 shows typical flow cytometry plots of CD4⁺ (upper figure) and CD8⁺ (lower figure) memory T cells generated by RBD-specific IL-2 in Test Example 5;
FIG. 19 shows flow cytometry plots of CD8⁺CD44⁺IL-2 cells in Test Example 5;
FIG. 20 shows flow cytometry plots of CD4⁺CD44⁺IL-2 cells in Test Example 5;
FIG. 21 shows flow cytometry plots of CD4⁺CD44⁺CD62L⁻ cells in Test Example 5;
FIG. 22 shows flow cytometry plots of CD8⁺CD44⁺CD62L⁻ cells in Test Example 5;
FIG. 23 shows typical flow cytometry plots of 6 T follicular helper cells in Test Example 6;
FIG. 24 shows flow cytometry plots of T follicular helper cells in Test Example 6;
FIG. 25 shows typical flow cytometry plots of plasmablasts in Test Example 6;
FIG. 26 shows flow cytometry plots of plasmablasts in Test Example 6;
FIG. 27 shows flow cytometry plots of germinal center B cells in Test Example 6;
FIG. 28 shows viral load in turbinate tissues of experimental mice in Test Example 7;
FIG. 29 shows viral load in trachea tissues of experimental mice in Test Example 7; and
FIG. 30 shows viral load in lung tissues of experimental mice in Test Example 7.

### DESCRIPTION OF THE EMBODIMENTS

At present, the study of sequential immunization with mRNA and protein vaccine is still blank.

The present invention provides a recombinant protein vaccine and an mRNA vaccine against infection by SARS-CoV-2 or a variant thereof, and the two vaccines are combined or used jointly. In a specific embodiment of the present invention, a protein vaccine RBD-HR/trimer is used as a third dose of heterologous booster after vaccination of two doses of mRNA vaccine.

Compared with a recombinant protein vaccine prepared with MF59-like adjuvant, a single dose of mRNA vaccine against infection by SARS-CoV-2 elicits superior germinal center (GC) responses. However, the present study found no significant difference in germinal center responses between three doses of homologous RBD-HR/trimer vaccine and homologous mRNA vaccine. Researchers have also found that, compared to those vaccinated with mRNA-1273, the mRNA-Omicron booster fails to elicit stronger humoral or cellular immune responses. In addition, the study also showed that the thermal stability and antigenicity of recombinant Omicron RBD were impaired. Therefore, in the study of the present invention, a recombinant RBD protein based on Delta variants instead of Omicron variants was designed to effectively achieve broad variant neutralization specifically against the Delta variants. Experimental verification showed that a heterologous recombinant protein vaccine booster provides stronger immunity and protection than a homologous mRNA vaccine.

The solution of the present invention will be explained with reference to embodiments. It will be understood by those skilled in the art that the following examples are merely intended for illustrating the present invention, instead of limiting the scope of the present invention. If specific technologies or conditions are not specified in the embodiments, technologies or conditions described in literatures in the art or product specifications should prevail. If manufacturers are not indicated in reagents or instruments used, they are all conventional products that can be obtained through market purchase.

### Embodiment 1 Preparation of mRNA vaccine and recombinant protein vaccine

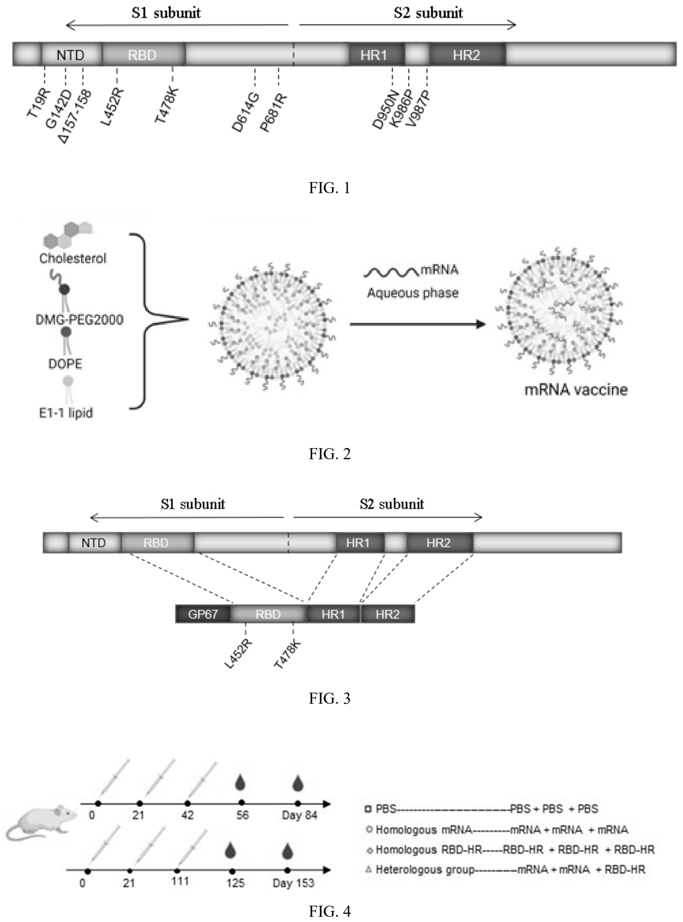

As shown in FIG. 1, based on the full-length S protein of Delta strains of SARS-CoV-2 variant strains, an mRNA vaccine capable of expressing the full-length spike protein was prepared, with an amino acid sequence shown in SEQ ID No.3 and an mRNA sequence shown in SEQ ID No.2 (FIG. 1). The mRNA vaccine was prepared by mixing oil phase (E1-1 lipid, DOPE, cholesterol, DMG-PEG2000 and ethanol) and water phase (mRNA sodium citrate solution) in a proper proportion (FIG. 2). The endotoxin of the mRNA vaccine was less than 15EU/dose or less than 30EU/mL.

A recombinant trimer vaccine with Delta-derived receptor binding domain (RBD) protein (including L452R and T478K) was developed. An RBD/HR trimer protein was prepared and mixed with an MF59-like adjuvant in equal volume to prepare an RBD-HR/trimer recombinant protein vaccine (FIG. 3). Given the well-established efficacy of the MF59-like adjuvant in the response between antibodies and T cells, the MF59-like adjuvant was selected as an adjuvant to enhance the immunogenicity of RBD-HR/trimer protein.

A specific preparation process includes:

### (1) Preparation of RBD/HR trimer recombinant protein vaccine:

### 1. Construction and design of RBD/HR trimer protein

The S protein of SARS-CoV-2 was a membrane-located protein. Therefore, in order to mimic its secretion process, the signal peptide sequence of GP67 was added to the N-terminal of the protein during the construction of S-RBD-HR protein expression in SARS-CoV-2 to assist in the secretion and expression of the protein, and this signal peptide would be spontaneously removed by insect cells in a protein secretion process. Meanwhile, thioredoxin (Trx) tag of *Spodoptera frugiperda* (*S. frugiperda*) was added after GP67 signal peptide to assist in S-RBD-HR folding, 6xhis tag was added to help subsequent purification, and an EK enzyme digestion site was added to remove both Trx and 6xhis tags. The construction and design of protein expression will be able to remove all non-S-RBD-HR redundant amino acids through EK enzyme digestion. The amino acid design sequence of the protein is shown in SEQ ID No.4

The expression vector of the S protein was constructed based on pFastBac1 vector (ampicillin resistance), inserted into pFast-bacI vector using BamHI and HindIII enzyme digestion sites, and optimized according to insect cell preferred codons.

### 2. Amplification of recombinant baculovirus

Based on the principle of bacterial transposon, site-specific transpositions were generated by BAC-to-BAC expression system via Tn7 transposon elements to complete the construction of recombinant bacmid in Escherichia coli (DH10Bac, including bacmid (kanamycin resistance) and helper plasmid (tetracycline resistance)). Recombinant bacmid was extracted and transfected into sf9 insect cells using Beyotime's Lipoinsect^{™} transfection reagent, to produce recombinant baculovirus capable of expressing the target gene. At 72-hour post-transfection, first generation virus was collected, P2 to P4 generation virus was amplified, and protein was expressed by P3 or P4 generation virus.

### 3. Protein expression

Hi5 insect cells (sf9 cells) were infected with P3 or P4 generation virus, with multiplicity of infection (MOI of 0.5-10). A supernatant was collected after cultured for 48-72 hours. An optimal harvest time may be different every time according to the virus amount and the cell state and it is generally appropriate to examine about 50% of cell lesions under a microscope.

### 4. Protein purification

The harvested culture supernatant was centrifuged at 4°C at a high speed and filtered by a 0.22um filter membrane, and a recombinant protein was preliminarily purified by the affinity purification method (Histrap nickel column). Then, the recombinant protein was purified by MonoQ ion column and Superdex 200 10/300GL molecular sieve, and the protein purity was identified by SDS-PAGE, and the purity required was over 95%. After purification by molecular exclusion chromatography, an RBD-HR/trimer protein antigen with good purity was obtained, which could be used for subsequent immune protection research. The purified recombination RBD-HR/trimer protein antigen was diluted at the 120µg/ml in PBS.

RBD-HR/trimer recombinant protein vaccine was prepared by mixing the MF59-like adjuvant and the RBD-HR/trimer protein in equal volume.

### (2) Preparation of mRNA vaccine

The mRNA vaccine capable of expressing the full-length spike protein was prepared by microfluidic technologies. E1-1 lipid, DOPE, cholesterol and DMG-PEG2000 were dissolved in absolute ethyl alcohol, and a specified volume of E1-1 organic phase solution with the concentration of 18mg/mL was prepared in the molar ratio of 50:10:38.5:1.5. Meanwhile, the mRNA expressing the full-length spike protein (SEQ ID No.2) was prepared into a specified volume of 0.6mg/mL with a citric acid buffer. An mRNA-LNP complex was prepared by microfluidic equipment with the flow rate ratio of water phase to organic phase of 3:1 and the total flow rate of 9mL/min. After the ethanol was removed by phosphate buffer (PBS) ultrafiltration, the mRNA vaccine capable of expressing the full-length spike protein was obtained.

The following experiments illustrate the beneficial effects of the present invention.

### Test Example 1 Mouse immune experiment

Female NIH mice aged 6-8 weeks were purchased from Charles River Laboratories, and the mice were raised in an environment without special pathogens. As shown in FIG. 4, NIH female mice were randomly divided into four groups: PBS group, homologous mRNA group (mRNA), homologous RBD-HR/trimer group (RBD-HR) and heterologous group (mRNA+RBD-HR).

Mice were administered in following either homologous or heterologous vaccination sequences. NIH mice were respectively immunized with 1µg, 5µg and 10µg mRNA vaccine on days 0 and 21, and a corresponding dose of mRNA or 10µg RBD-HR vaccine on day 42. As a control group, mice were respectively vaccinated with three doses of PBS, mRNA and RBD-HR/trimer vaccine of the same type and dose. In other words, each mouse in the homologous mRNA group was vaccinated with 1µg or 5µg or 10µg mRNA vaccine three times, while each mouse in the homologous RBD-HR group was vaccinated with 10µg RBD-HR vaccine three times. The heterologous group was vaccinated with the same dose of mRNA vaccine for the first two times and 10µg RBD-HR vaccine for the third time.

In order to test the effect of RBD-HR vaccine on immune response for a long time after two doses of mRNA vaccine, we immunized mice with the third dose of booster on day 111. Blood samples were collected through orbital vein on days 56, 84, 125 and 153, and centrifuged at 6000 rpm for 10 minutes at 4°C. Serum samples were stored at -20°C before use. Seven days after third immunization, the mice were killed and the spleen and lymph nodes were taken. Spleen tissues and lymph nodes were homogenized, and a single-cell suspension was prepared with a lymphocyte separation medium.

### Test Example 2 Detection of specific antibodies against RBD

An enzyme-linked immunosorbent assay (ELISA) was used to detect RBD specific antibodies in serums. A 96-well NUNC-MaxiSorp plate (Thermo Fisher Scientific, USA) was coated with a recombinant RBD protein (0.1µg/well) at 4°C for 12 hours. The plate was washed three times with 1× PBST (1× PBS + 0.1% Tween-20), followed by blocking with 1% bovine serum albumin (BSA) at 37°C for 1 hour. The plate was incubated with serum samples at two-fold dilutions for 1 hour at room temperature and washed three times with 1× PBST. Horseradish peroxidase (HRP)-goat anti-mouse IgG antibodies diluted at 1:10000 were added to the plate, incubated for 1 hour at room temperature and washed five times. 3,3', 5,5'-tetramethylbiphenyl diamine (TMB) was added to the plate and incubated in the dark for 10 minutes. Such incubation was stopped with 1 M H₂SO₄ (100µl/well), and the absorbance was measured at 450nm.

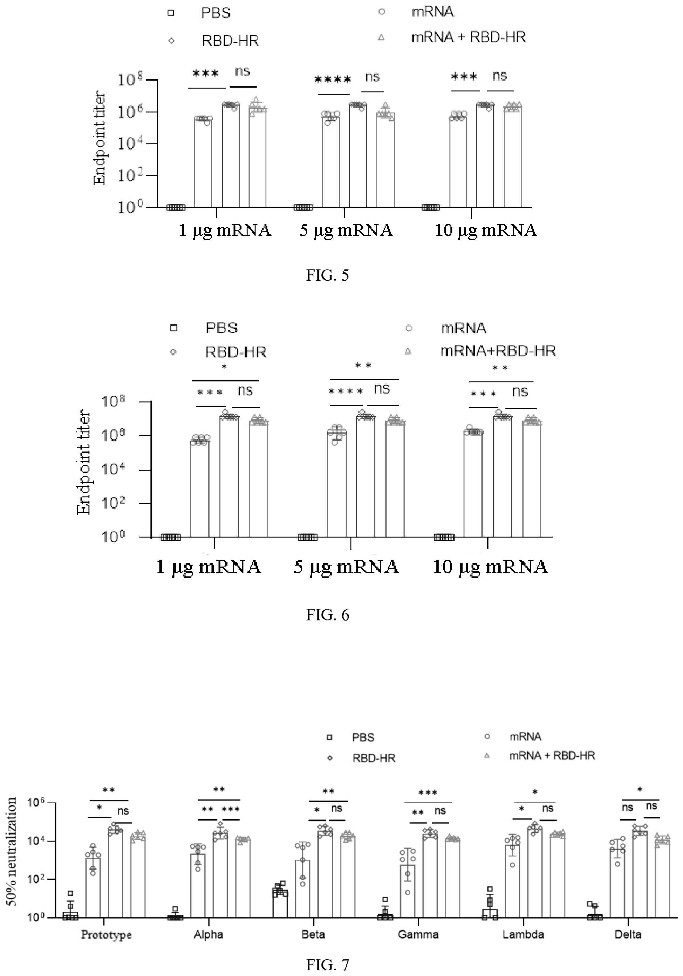

As shown in FIG. 5 and FIG. 6, in an immunization program of a short time interval (immunization on days 0, 21 and 42) (FIG. 5) and a long time interval (immunization on days 0, 21 and 111) (FIG. 6), a heterologous immunization regimen elicited higher levels of anti-RBD binding antibodies than the homologous immunization of three doses of mRNA vaccine.

### Test Example 3 Pseudovirus neutralization test

In order to evaluate whether a heterologous vaccine booster has better protection effect than a homologous vaccine booster, mice in a homologous mRNA group were injected with 1µg mRNA vaccine each time, and serum was collected on days 84 and 153 (long-term immunization) after first immunization to conduct pseudovirus neutralization test.

SARS-CoV-2 pseudovirus (GFP-Luciferase), BA.3 and BA.4/5 pseudoviruses and other SARS-CoV-2 variant strain pseudoviruses were purchased from Vazyme Biotech Co., Ltd. (China) and Genomeditech (China). NIH mice were injected with mRNA vaccine intramuscularly. After inactivation at 60°C for 30 minutes, the serum was diluted with triple gradient of complete dimethyl ether medium. Diluted serum and luciferase pseudovirus (wild type, B.1.1.7, B.1.351, P.1, B.1.617, C.37, BA.1, BA.2, BA.2.12.1, BA.3, BA.4/5) were incubated at 37°C for 1 hour. 1.2×104 293T/ACE2 cells were added to each well to express a reporter gene. After 48 hours, the supernatant of infected cells was removed, then 100µL of fluorescein substrate (50µL PBS+50µL fluorescein substrate) premixed with PBS 1:1 and equilibrated to room temperature was added to each well and incubated for approximately 2 minutes. Finally, chemiluminescence was detected by a fluorescence analyzer.

As shown in FIG. 7, in a short-interval immunization program, the heterologous sequential immunization group elicited a higher level of broad-spectrum neutralizing antibodies compared to the homologous mRNA vaccine immunization group.

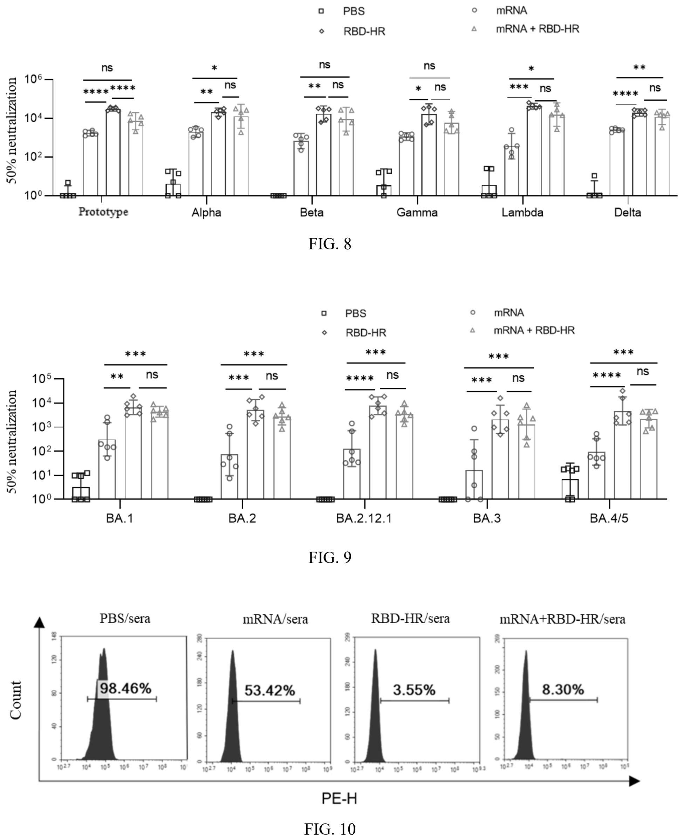

As shown in FIG. 8, in a long-interval immunization program, the heterologous immunization group also elicited a higher broad-spectrum neutralizing antibody titer compared to the homologous mRNA vaccine group.

As shown in FIG. 9, in the long-interval immunization program, the heterologous immunization group could elicit a higher neutralizing antibody level against Omicron and variant strains thereof, including BA.4/5 variant strains.

### Test Example 4 Blocking assay of binding between RBD protein and ACE2 receptor

Binding to an ACE2 receptor was detected by an RBD-Fc (Omicron) protein. The RBD-Fc protein (0.3µg/mL) was continuously diluted into immune serums (the immune dose of an mRNA vaccine was 1µg/time/animal, and the immune dose of protein was 10µg/time/animal) and incubated at 37°C for 30 minutes. Then, 293T/ACE2 cells (5×10⁴) were added and incubated at room temperature for 30 minutes. The cells were washed three times with BPBS (1% BSA was added to PBS). The anti-human IgG-Fc antibody labeled with pe was added to the cells and incubated in the dark at 4°C for 30 minutes. A binding concentration was determined by a flow cytometer, and the results were analyzed by NovoCyte flow cytometer (ACEA Biosciences).

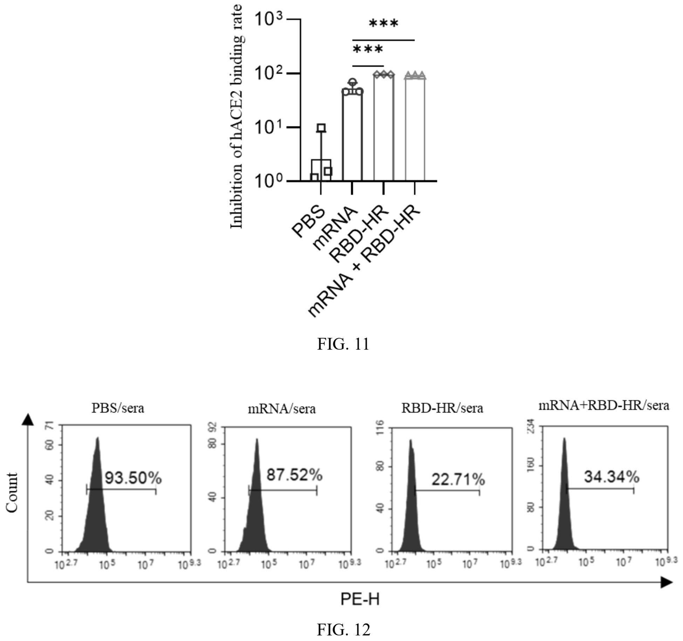

As shown in FIG. 10 and FIG. 11, in the 21-day interval immunization program, a third dose of heterologous RBD/HR trimer booster injection could effectively enhance the binding ability of mouse serum to block hACE2 and improve the blocking rate.

As shown in FIG. 12 and FIG. 13, 90 days after second-dose immunization, in the immunization program where the third dose of booster injection was immunized, the third dose of the booster injection of the heterologous RBD/HR trimer vaccine could also effectively enhance the binding ability of mouse serum to block hACE2 compared with the third dose of homologous mRNA vaccine and improve the blocking rate.

To sum up, in order to verify the potential mechanism of antiviral neutralization, we used flow cytometry to detect the blocking of the binding between the ACE2 receptor and the RBD-Fc (Omicron) on cell surfaces. Consistent with the pseudovirus assay results from two immunization regimens with different time intervals, heterologous immunization also demonstrated that the inhibition rate between the RBD-Fc (Omicron) and the human ACE2 receptor was higher than that of homologous mRNA immunization. Notably, the shorter regimen has a higher inhibition rate, which emphasizes the importance of receiving supplementary immunization at an appropriate time. These results indicate that the heterologous vaccine including protein subunits may be a better strategy to elicit stronger humoral immunity than the homologous vaccine of mRNA vaccine.

### Test Example 5 Flow cytometry detection of splenic lymphocytes

Spleen lymphocytes were cultured in a 1640 culture medium for 72 h and subjected to intracellular cytokine staining (ICS). 10% fetal bovine serum, 100µg/mL streptomycin, 100U/mL penicillin, 1 mM pyruvic acid (all which were purchased from Gibco Company, USA), 50µM β-mercaptoethanol and 20U/mL IL-2 (all which were purchased from Sigma-Aldrich Company) were added to the 1640 culture medium. In addition, RBD (10µg/well) was added to viable cells. To block intracellular cytokine secretion, the cells were incubated with brefeldin A (BFA, BD Biosciences) for 6 hours prior to staining. The cells were washed in 1 × PBS coolant and stained for 30 minutes at 4°C using CD4 (Brilliant Violet 421), CD8 (FITC), CD44 (Brilliant Violet 510), B220 (PerCP/Cyanine5.5), and MHC-II (BV711) (all which were purchased from BioLegend Company). Then, the cells were fixed and permeabilized to facilitate intracellular staining with anti-IFN-γ (PE), anti-IL-4 (Brilliant Violet 421) and IL-2 (APC) (all flow antibodies were from BioLegend Company) for 2 hours at room temperature. The cells were washed with cold 1×PBS and then detected by flow cytometry.

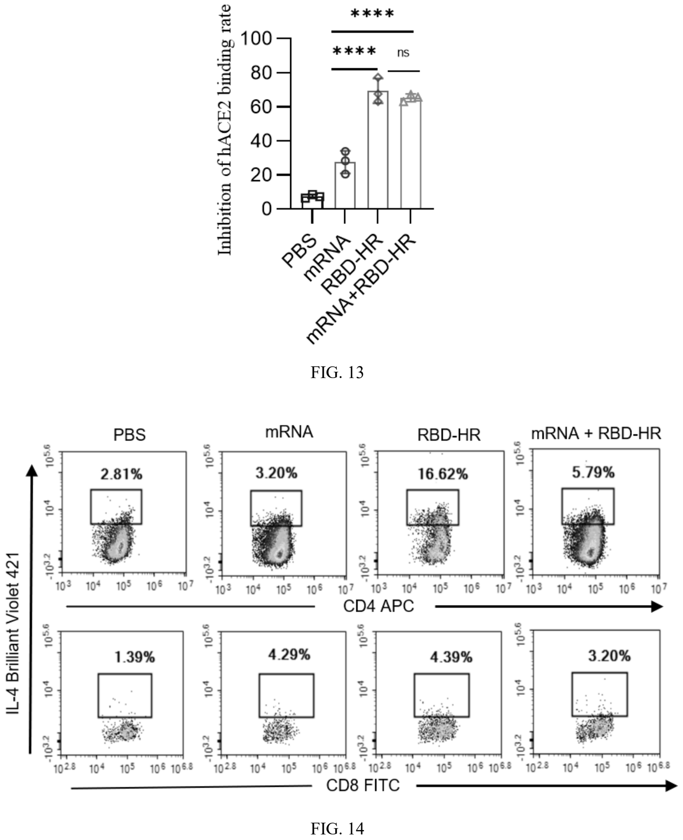

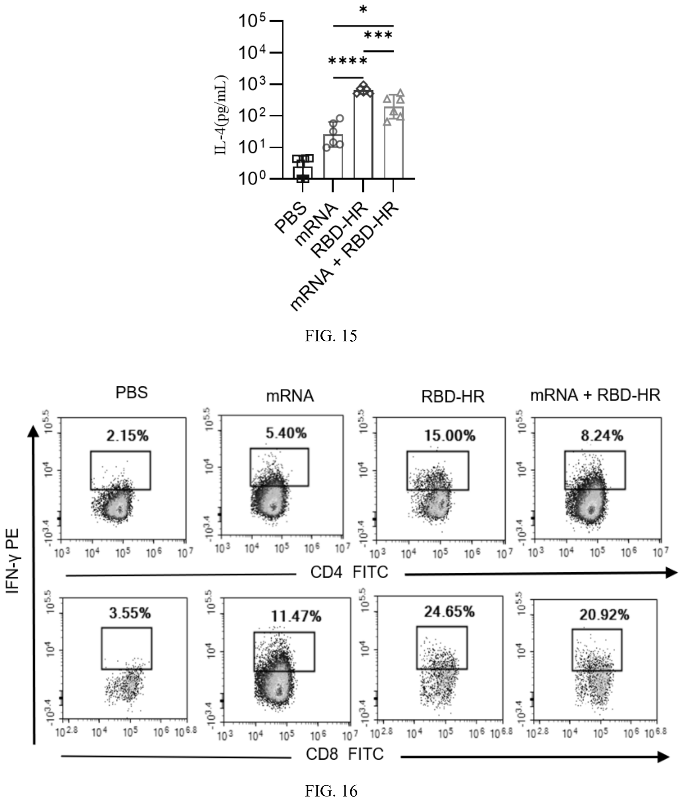

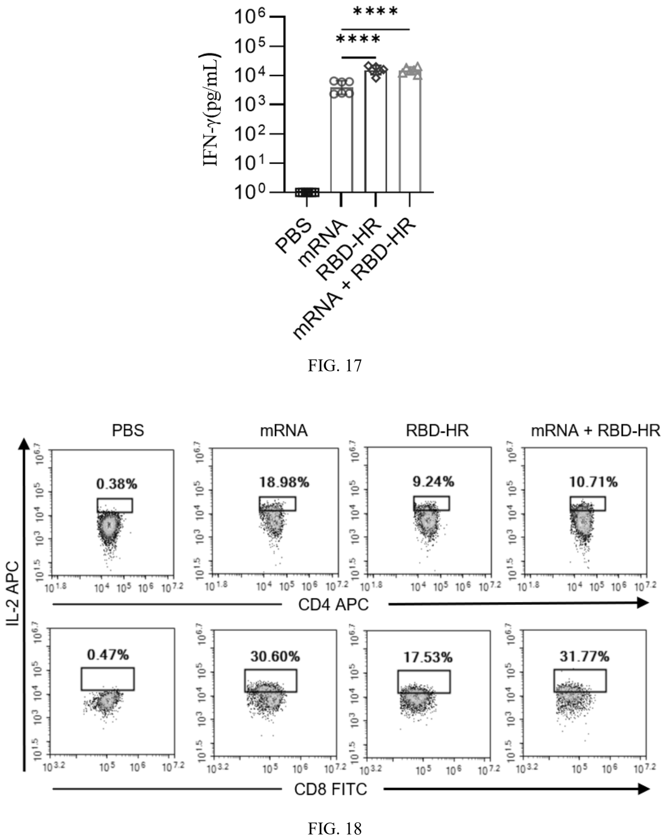

T-cell response is a primary determinant of clinical outcomes and plays an important role in SARS-CoV-2 prevention. Mice in the homologous mRNA group were intramuscularly injected with 5µg mRNA each time. The response of CD4⁺ and CD8⁺ T cell to SARS-CoV-2 RBD was assessed. Single-cell suspensions of splenic lymphocytes were prepared to evaluate the boosting effect of RBD-HR/trimer heterogenous vaccine on cellular immune response. After stimulation with RBD protein at 37°C for 72 hours, secreted cytokines (IL-4 and IFN-γ) were detected by the flow cytometer and ELISA. Higher levels of IL-4 (FIG. 14 and FIG. 15) and IFN-γ (FIG. 16 and FIG. 17) were observed in the heterologous vaccine group and the homologous protein vaccine group, indicating that the RBD-HR/ trimer vaccine could elicit a strong T-cell response.

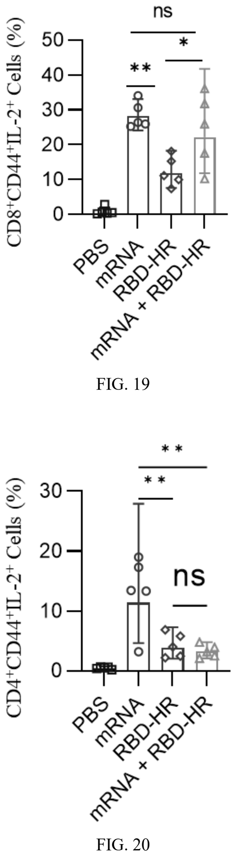

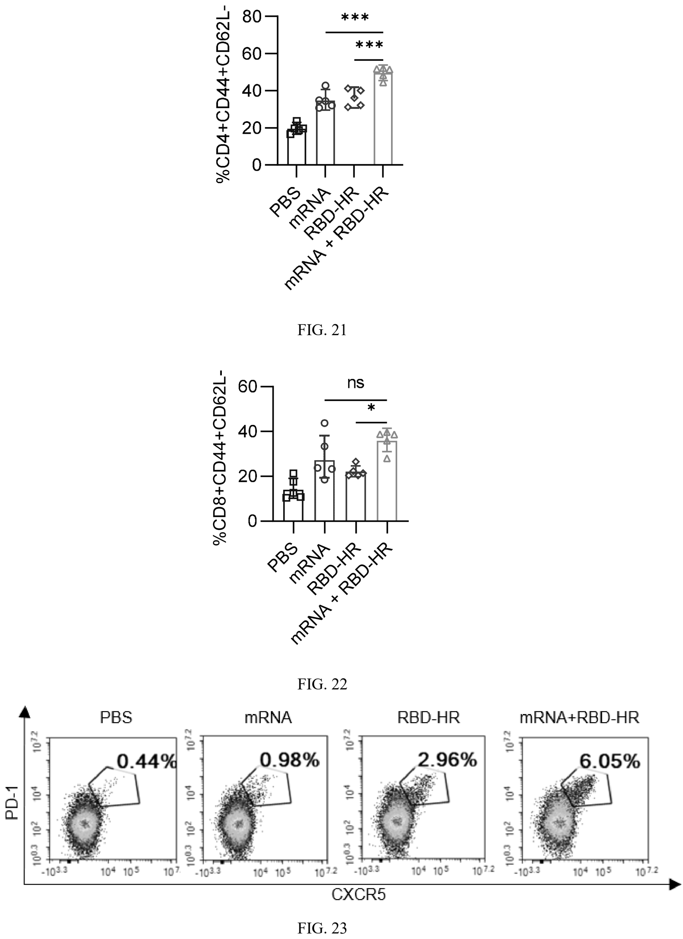

It was reported that mRNA vaccines could cause solid T cell responses associated with IL-2, whereas protein vaccines failed to do so. Notably, both heterologous vaccination and homologous mRNA vaccination elicited comparable IL-2-associated CD8⁺ T cell responses (FIG. 18 to FIG. 20). Memory T cell responses were crucial for accelerating and enhancing immune responses and became even more important especially reinfection with same pathogens. In addition, immune T cell memory response could also provide protective immunity against a variety of variants. On day 49, we evaluated subsets of splenic effector memory (CD44⁺CD62L⁻) T cells. The numbers of both CD4⁺ and CD8⁺ effector memory T cells were higher than those in the homologous mRNA and RBD-HR/trimer vaccination groups (FIG. 21 and FIG. 22). To sum up, the cellular immunity elicited by the heterologous RBD-HR/trimer vaccination is stronger than that elicited by the homologous mRNA vaccination.

### Test Example 6 Detection of splenic memory T cells

After the single-cell suspensions of splenic lymphocytes were prepared, we used CD3 (PerCP/Cyanine 5.5), CD62L (Brilliant Violet 421), CD4 (APC), CD8 (FITC), CD69 (PE-cy7), and anti-mouse CD44 (Brilliant Violet) 510), CD95 (FITC), B220 (PE-cy7), GL7 (PE), CD4 (Brilliant Violet 510), CD8 (Brilliant Violet 510), CD19 (Brilliant Violet 421), CD138 (APC), CXCR5 (APC), PD1 (Brilliant Violet 421) and anti-mouse CD19 (PE) to stain Tfh cells, GC B cells, plasmablasts and effector memory T cells (all flow antibodies were from BioLegend Company). The cells were stained at 4°C in the dark. After 30 minutes, the cells were washed with 1×PBS and detected by a flow cytometer.

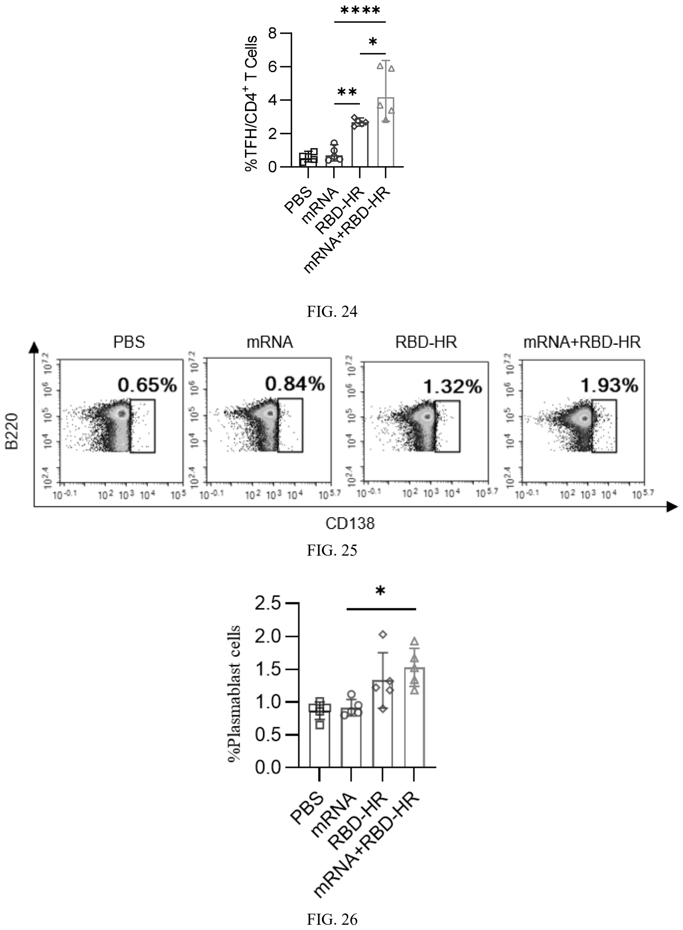

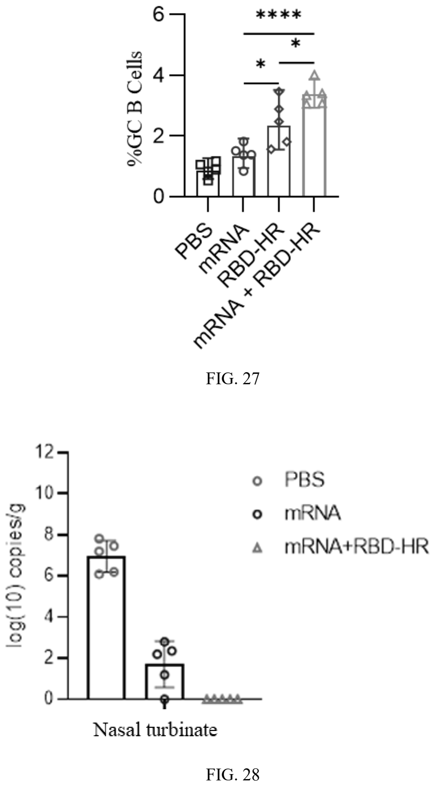

Long-lasting memory response provides rapid and effective protective immunity upon reinfection. Mice in the homologous mRNA group were intramuscularly injected with 5µg mRNA each time. To evaluate whether heterologous vaccination could elicit further memory response of RBD-HR/trimer, we analyzed the formation of T follicular helper (Tfh) cell effect, plasmablast responses and germinal center (GC) effect, which are very important for long-lasting memory response. In addition, GC B cells and Tfh cells are closely associated with the neutralizing antibody response. The results showed that heterologous vaccination with RBD-HR/trimer could enhance the responses of Tfh (FIG. 23 and FIG. 24), plasmablast (FIG. 25 and FIG. 26) and GC-B (FIG. 27). Notably, the heterologous vaccination group exhibited the highest frequencies of Tfh and GC B cells. Therefore, these results indicate that the heterologous RBD-HR/trimer vaccine can elicit a greater long-lasting memory immune response than the mRNA vaccine booster.

### Test Example 7 Live-virus challenge assay

Mice were administered in following either homologous or heterologous vaccination sequences.

Mice were immunized with 5µg mRNA vaccine on days 0 and 21, and the corresponding dose of 5µg mRNA or 10µg RBD-HR vaccine respectively on day 42. A PBS group was taken as a control group.

To test the protective efficacy of the RBD-HR vaccine on mice primed with two doses of mRNA vaccine, live virus challenge was conducted on day 56 via intranasal vaccination. All experimental animals were challenged with Omicron (BA.1) via intranasal vaccination, and the dose of challenge was 1×10⁶ TCID50 per mouse. On day 4 after challenge, the right lung lobe, nasal turbinates and tracheal tissues of the mice were dissected for viral load detection. The tissues were cut into small pieces, mixed and weighed. Nearly 100mg tissues were weighed and homogenized with 800µL Trizol. 400µL tissue homogenate was taken to extract RNA, and RNA was finally dissolved in 50µL nuclease-free solution and stored at -80°C for future use. RNA was used for the subsequent detection of viral load by qRT-PCR (one-step method). The genomic RNA (gRNA) of the virus was detected by real-time fluorescence quantitation PCR (qRT-PCR). Primers and probes adopted targeted the N gene of SARS-CoV-2. Positive: SEQ ID No.6 5'-GACCCCAAAATCAGCGAAAT-3'; reverse: SEQ ID No.7 5'-TCTGGTTACTGCCAGTTGAATCTG-3'; probe: SEQ ID No.8 5'-FAM-ACNCCGCATTACGTTTGGTGGACC-BHQ1-3'.

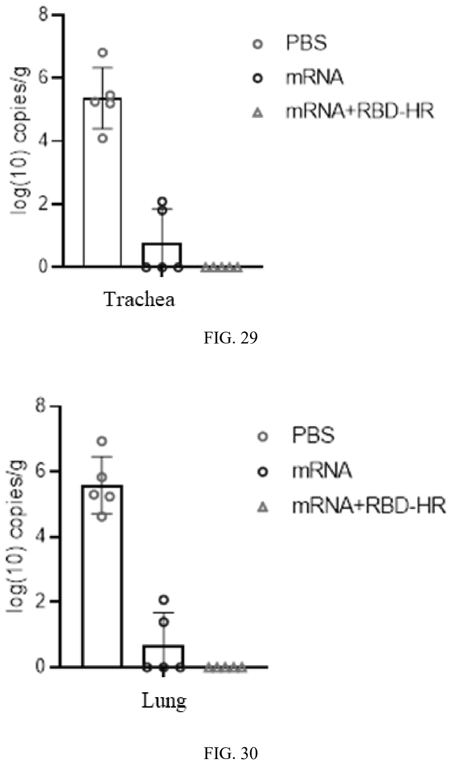

Results showed that high levels of viral load were detected in nasal turbinates, trachea, and lung tissues of experimental mice in the PBS group (FIG. 28 to FIG. 30). The mRNA vaccine group exhibited significantly reduced viral loads, with only low levels of viral residues. No viral load was detected in any tissue of the RBD-HR sequential mRNA vaccine group. This demonstrates that in mice, the RBD-HR sequential mRNA vaccine can better inhibit the replication of the virus and eliminate the residual virus.

To sum up, in the present invention, a third-dose heterologous booster of self-assembled recombinant RBD-HR vaccine and two doses of mRNA vaccines can significantly enhance humoral and cellular immune responses, showing higher anti-RBD IgG levels, stronger neutralizing antibodies and IFN-γ-secreting T cells. In addition, heterologous vaccination can elicit a stronger effector memory response than homologous RBD-HR and mRNA vaccination. In addition, the lymphoblastic response elicited by heterologous vaccination is higher than that of the homologous mRNA group, and the responses of GC-B cells and Tfh elicited are higher than those of the two groups. This indicates that heterologous vaccination could elicit a higher long-lasting memory response. Compared with mRNA homologous sequencing, RBD-HR heterologous sequencing provides better protective effects. Therefore, the present invention provides a new strategy for heterologous enhanced immunity. A third heterologous protein subunit booster (RBD-HR/trimer vaccine) is a highly suitable candidate following two doses of the mRNA vaccines.

## Claims

1. An mRNA vaccine for the prevention and/or treatment of SARS-CoV-2 or its mutant infections, **characterized in that** it contains an mRNA vaccine against SARS-CoV-2 mutant infections as the active ingredient.

2. A recombinant protein vaccine for preventing and/or treating infection by SARS-CoV-2 or a variant thereof, **characterized in that** it comprises a recombination protein vaccine as the active component against infection by SARS-CoV-2 variants.

3. The mRNA vaccine according to claim 1, wherein an mRNA sequence comprises a full-length nucleic acid sequence encoding the S protein of the SARS-CoV-2 variants.

4. The mRNA vaccine according to claim 1, wherein the full-length nucleic acid sequence encoding the S protein of the SARS-CoV-2 variants is shown in SEQ ID No.1 or/and SEQ ID No.2 sequence, or is a variant that exhibits homology and same or similar biological activity with the SEQ ID No.1 or/and SEQ ID No.2 sequence and is obtained by substitution and/or deletion and/or insertion of at least one amino acid in the SEQ ID No.1 or/and SEQ ID No.2 sequence; preferably, a variant sequence is a variant obtained by substitution and/or deletion and/or insertion of 1-400 amino acids in the SEQ ID No.1 or/and SEQ ID No.2 sequence; and preferably, the variant sequence is a variant obtained by substitution and/or deletion and/or insertion of 5-30 amino acids in the SEQ ID No.1 sequence.

5. The recombinant protein vaccine according to claim 2, wherein a protein and/or a protein precursor comprises a protein having at least one RBD sequence and at least one HR sequence derived from the S protein of SARS-CoV-2 or the variant thereof.

6. The recombinant protein vaccine according to claim 2, wherein a protein and/or a protein precursor comprises an amino acid sequence shown in SEQ ID No.4 sequence, or comprises a variant that exhibits homology and same or similar biological activity with the SEQ ID No.4 sequence and is obtained by substitution and/or deletion and/or insertion of at least one amino acid in the SEQ ID No.4 sequence; preferably, an RBD sequence is a variant obtained by substitution and/or deletion and/or insertion of 1-400 amino acids in the SEQ ID No.4 sequence; and preferably, the RBD sequence is a variant obtained by substitution and/or deletion and/or insertion of 5-30 amino acids in the SEQ ID No.4 sequence.

7. A composition against infection by SARS-CoV-2 or a variant thereof, comprising a preparation with an mRNA vaccine and a recombinant protein vaccine as active components, wherein the mRNA vaccine is the mRNA vaccine according to claim 1, 3 or 4, and the recombinant protein vaccine is the recombinant protein vaccine according to claim 2, 5 or 6.

8. A combined drug against infection by SARS-CoV-2 or a variant thereof, comprising an mRNA vaccine and a recombinant protein vaccine against SARS-CoV-2 or the variant thereof, wherein the mRNA vaccine and the recombinant protein vaccine are administered respectively, simultaneously or sequentially, the mRNA vaccine is the mRNA vaccine according to claim 1, 3 or 4, and the recombinant protein vaccine is the recombinant protein vaccine according to claim 2, 5 or 6.

9. The mRNA vaccine according to claim 1 or the recombinant protein vaccine according to claim 2 or the combined drug according to claim 8, wherein the mRNA vaccine, the recombinant protein vaccine or the combined drug is formulated as intramuscular injections, nasal drops, sprays, nasal sprays, or inhalants.

10. The combined drug according to claim 8, wherein a protein formed by the RBD sequence and the HR sequence in a recombinant S protein spontaneously forms a trimer.

11. The combined drug according to claim 8, wherein a homologous amino acid sequence is selected from at least one of RBD sequence of Alpha, Beta, Gamma, Delta, Omicron.

12. The combined drug according to claim 10 **characterized in that**: the protein precursor is linked with a signal peptide and/or a protein tag on the protein that resists SARS-CoV-2 or its mutant infection; preferably, the signal peptide comprises a native signal peptide of S protein or a variant thereof and/or a human tPA signal peptide additionally appended before the native signal peptide; preferably, the protein tag is selected from at least one of a histidine tag, a thioredoxin tag, a glutathione transferase tag, a ubiquitin-like modifier protein tag, a maltose-binding protein tag, a c-Myc protein tag and an Avi tag protein tag; and more preferably, the protein tag is a Trx tag and/or a 6His tag.

13. The combined drug according to claim 8, wherein a protease recognition site for excising the protein tag is further linked to the protein against infection by SARS-CoV-2 or the variant thereof; and preferably, the protease is selected from at least one of enterokinase, TEV protease, thrombin, coagulation factor Xa, carboxypeptidase A and rhinovirus 3c protease.

14. Host cells used in the recombinant protein vaccine according to claim 2, wherein the host cells are selected from at least one of insect cells, mammalian cells, Escherichia coli and yeast; preferably, the insect cells are selected from at least one of sf9 cells, sf21 cells and Hi5 cells; and preferably, the mammalian cells are either CHO cells or HEK293 cells.

15. The mRNA vaccine according to claim 1 or the recombinant protein vaccine according to claim 2, wherein the mRNA vaccine and/or the recombinant protein vaccine further comprises a pharmaceutically acceptable excipient or adjuvant component.

16. The combined drug according to claim 13, wherein the recombinant protein vaccine further comprises a pharmaceutically acceptable excipient or adjuvant component; the adjuvant component is an immunologic adjuvant; preferably, the immunologic adjuvant is selected from at least one of the following: squalene-based oil-in-water emulsion, aluminum salt, calcium salt, phytosaponin, phytopolysaccharide, monophosphate lipid A, muramyl dipeptide, muramyl tripeptide, bacterial toxin, GM-CSF cytokine, lipid and cationic liposomal material; further, the immunologic adjuvant satisfies at least one of the following: the squalene-based oil-in-water emulsion being MF59, the aluminum salt being selected from at least one of aluminum hydroxide and alum, the calcium salt being tricalcium phosphate, the phytosaponin being either QS-21 or ISCOM, the phytopolysaccharide being astragalus polysaccharide, the bacterial toxin being selected from at least one of recombinant cholera toxin and diphtheria toxin, the lipid being selected from at least one of the following: phosphatidylethanolamine, phosphatidylcholine, cholesterol, dioleoylphosphatidylethanolamine; the cationic liposome material being selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl-2,3-dioleyloxypropyl-2-(2-spermidinecarboxamido)ethyl ammonium trifluoroacetate, trimethyldodecylammonium bromide, trimethyltetradecylammonium bromide, trimethylhexadecylammonium bromide, dimethyldioctadecylammonium bromide, and CpG ODN.

17. Use of the mRNA vaccine according to any one of claims 1, 3, 4 and 9, the recombinant protein vaccine according to any one of claims 2, 5, 6 and 9, the composition according to claim 7 or the combined drug according to any one of claims 8, 9-13 in preparing drugs for preventing and/or treating infection by SARS-CoV-2 or a variant thereof.

18. Use of the mRNA vaccine according to any one of claims 1, 3, 4 and 9, the recombinant protein vaccine according to any one of claims 2, 5, 6 and 9, the composition according to claim 7 or the combined drug according to any one of claims 8, 9-13 in preparing drugs for treating and/or preventing infection or pathogenicity by SARS-CoV-2 variant strains, wherein the SARS-CoV-2 variant strains comprise at least one of Alpha, Beta, Gamma, Delta and Omicron.
